# FASCICULE DE BREVET EUROPEEN

(11) **EP 1 598 333 B1**
(45) Date de publication et mention de la délivrance du brevet: **18.10.2006**
(21) Numéro de dépôt: 05290384.6
(22) Date de dépôt: 21.02.2005
(51) Int. Cl.: C07C 213/10, C07C 217/56, C07B 57/00

(54) **Procédé de synthèse du (1S)-4,5-diméthoxy-1-(méthylaminométhyl)-benzocyclobutane et de ses sels d'addition, et application à la synthèse de l'ivabradine et de ses sels d'addition à un acide pharmaceutiquement acceptable**
Verfahren zur Herstellung von (1S)-4,5-Dimethoxy-1-(methylaminomethyl)-benzocyclobutan und deren Säureadditionssalze, sowie ihre Verwendung für die Synthese von Ivabradin und deren Säureadditionssalze von pharmazeutischen verträglichen Säure
Process for the preparation of (1S)-4,5-dimethoxy-1-(methylaminomethyl)-benzocyclobutane, acid addition salts thereof and its use for the synthesis of ivabradine and for its pharmaceutically acceptable acid addition salts

(30) Priorité: 19.05.2004 FR 0405453
(43) Date de publication de la demande: 23.11.2005
(73) Titulaire: Les Laboratoires Servier, 92415 Courbevoie Cedex (FR)
(72) Inventeur: Lerestif, Jean-Michel, 76190 Yvetot (FR); Gonzalez-Blanco, Isaac, 45002 Toledo (ES); Lecouve, Jean-Pierre, 76600 Le Havre (FR); Brigot, Daniel, 76190 Sainte-Marie-des-Champs (FR)

(56) Documents cités:
- EP-A- 0 534 859
- EP-A- 0 776 883
- KENNETH D. PAULL ET AL.: "A facile synthesis of 4-substituted 3a,4,5,9b-tetrahydrobenz(e)isoindolines" JOURNAL OF ORGANIC CHEMISTRY., vol. 37, no. 21, 1972, pages 3374-3376, XP002312221 USAMERICAN CHEMICAL SOCIETY, WASHINGTON, DC.

## Description

La présente invention concerne un procédé de synthèse du (1*S*)-4,5-diméthoxy-1-(méthylaminométhyl)-benzocyclobutane et de ses sels d'addition, et son application à la synthèse de l'ivabradine et de ses sels d'addition à un acide pharmaceutiquement acceptable.

Plus spécifiquement, la présente invention concerne un procédé de synthèse du composé de formule (I) de configuration (*S*) : 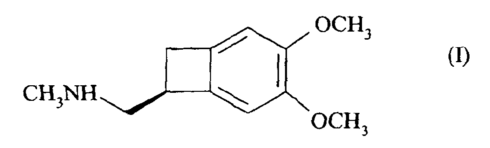 et de ses sels d'addition à un acide.

Le composé de formule (I) obtenu selon le procédé de l'invention est utile dans la synthèse de l'ivabradine de formule (II) : 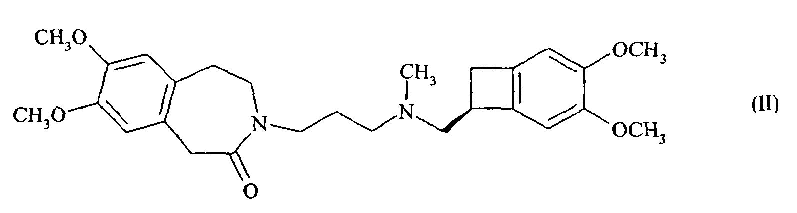 ou 3-{3-[{[(7S)-3,4-diméthoxybicyclo[4.2.0]octa-1,3,5-trién-7-yl]méthyl}(méthyl)amino] propyl}-7,8-diméthoxy-1,3,4,5-tétrahydro-2*H*-3-benzazépin-2-one,
de ses sels d'addition à un acide pharmaceutiquement acceptable et de ses hydrates.

L'ivabradine, ainsi que ses sels d'addition à un acide pharmaceutiquement acceptable, et plus particulièrement son chlorhydrate, possèdent des propriétés pharmacologiques et thérapeutiques très intéressantes, notamment des propriétés bradycardisantes, qui rendent ces composés utiles dans le traitement ou la prévention des différentes situations cliniques d'ischémie myocardique telles que l'angine de poitrine, l'infarctus du myocarde et les troubles du rythme associés, ainsi que dans les différentes pathologies comportant des troubles du rythme, notamment supra-ventriculaires.

La préparation et l'utilisation en thérapeutique de l'ivabradine et de ses sels d'addition à un acide pharmaceutiquement acceptable, et plus particulièrement de son chlorhydrate, ont été décrits dans le brevet européen EP 0534 859.

Ce brevet décrit la synthèse du composé de formule (I) par réduction du nitrile racémique de formule (III) : 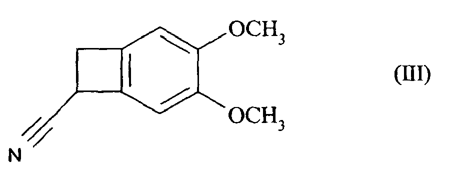 par BH₃ dans le tétrahydrofurane, suivie de l'addition d'acide chlorhydrique, pour conduire au chlorhydrate de l'amine racémique de formule (IV) : 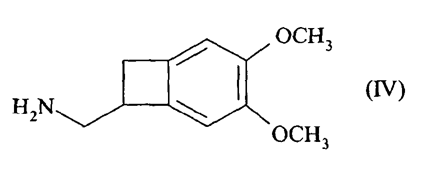 qui est transformé en carbamate de formule (V) : 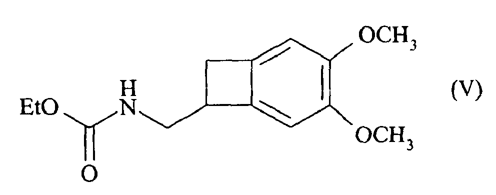 avant d'être réduit en amine méthylée de formule (VI) : 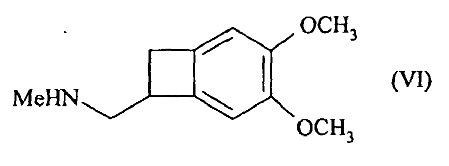 dont le dédoublement à l'aide d'acide camphosulfonique conduit au composé de formule (I).

Cette méthode a l'inconvénient de ne conduire au composé de formule (I) qu'avec un rendement très faible de 2 à 3%.

Ce rendement très faible est dû au faible rendement (4 à 5%) de l'étape de dédoublement de l'amine secondaire de formule (VI).

Or, compte-tenu de l'intérêt pharmaceutique de l'ivabradine et de ses sels, il était impératif de pouvoir accéder au composé de formule (I) avec un procédé industriel performant, et notamment avec un bon rendement et une excellente pureté chimique et énantiomérique.

La Demanderesse a trouvé que, de façon surprenante, il était bien plus avantageux d'effectuer le dédoublement sur l'amine primaire de formule (IV).

L'accès à l'amine de formule (IV) est décrit dans la publication J. Org. Chem. 1972, 37(21), 3374-3376. Cependant, aucun procédé de dédoublement de l'amine n'est mentionné dans ce document. D'autre part, EP 0 776 883 décrit l'utilisation de l'acide N-acétyl-L-glutamique comme agent de dédoublement; mais les benzylamines substituées ainsi dédoublées sont très différentes de l'amine de formule (IV).

Plus spécifiquement, la présente invention concerne un procédé de dédoublement de l'amine de formule (IV) : 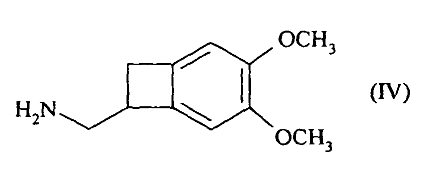 par réaction avec l'acide N-acétyl-L-glutamique,
suivie de la filtration ou de l'essorage de la suspension ainsi obtenue,
pour conduire au composé de formule (VII) : 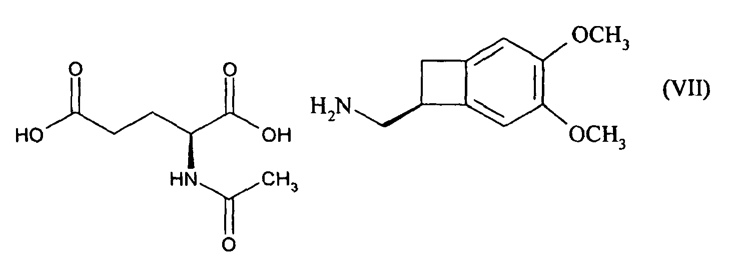 que l'on met en réaction avec une base, pour conduire à l'amine correspondante de formule (VIII), de configuration (S) :

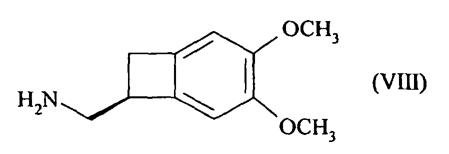

La réaction de l'amine de formule (IV) avec l'acide N-acétyl-L-glutamique est préférentiellement effectuée dans un solvant organique, préférentiellement un solvant alcoolique, seul ou en mélange avec un autre solvant organique, avec de l'eau ou avec un autre solvant organique et de l'eau.

La base utilisée pour transformer le sel de formule (VII) en amine de formule (VIII) est préférentiellement la soude.

Pour améliorer la pureté énantiomérique du composé de formule (VIII) ainsi obtenu, on peut ajouter une ou plusieurs étapes de recristallisation du composé de formule (VII) dans un solvant organique, préférentiellement un solvant alcoolique, seul ou en mélange avec un autre solvant organique, avec de l'eau ou avec un autre solvant organique et de l'eau, avant d'effectuer le retour base.

De façon inattendue, parmi les agents de dédoublement testés, seul l'acide N-acétyl-L-glutamique a permis d'obtenir l'amine de configuration (S) avec une excellente sélectivité, comme le montre le tableau suivant :

| **Agent de dédoublement** | **Nature de l'acide** | **Rapport (S) / (R)** |
|---|---|---|
| Acide (R)-camphosulfonique | Monoacide | pas de précipitation |
| (R)-acétyl phényl glycine | Monoacide | 55/45 |
| N-acétyl-D-valine | Monoacide | 55/45 |
| N-acétyl-L-leucine | Monoacide | 50/50 |
| **Acide N-acétyl-L-glutamique** | **Diacide** | **93/7** |

*Conditions* : solvant : éthanol 95%. Tous les sels ont été chauffés au reflux jusqu'à dissolution puis laissés recristalliser une nuit.

Le dédoublement de l'amine de formule (IV) est préférentiellement effectué dans un mélange éthanol/eau ou acétate d'éthyle/éthanol/eau.

La présente invention concerne également un procédé de synthèse du composé de formule (I), de configuration (S) : 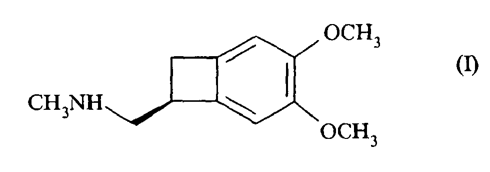 caractérisé en ce que l'on réduit le composé de formule (III) : 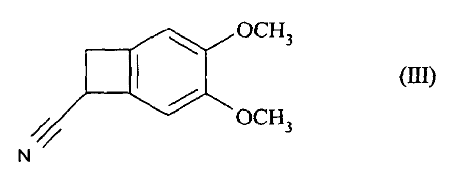 pour conduire à l'amine racémique de formule (IV), que l'on dédouble selon le procédé précédemment décrit, pour conduire à l'amine optiquement active de formule (VIII), que l'on met en réaction avec le chloroformiate d'éthyle, pour conduire au composé optiquement actif de formule (IX) : 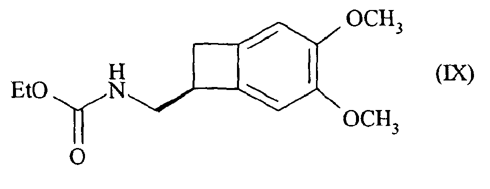 dont on réduit la fonction carbamate,
pour conduire au composé de formule (I).

L'addition d'acide conduit ensuite au sel correspondant.
En particulier, l'addition d'acide chlorhydrique gazeux ou d'acétate d'éthyle chlorhydrique conduit au chlorhydrate du composé de formule (I).

Le chlorhydrate du composé de formule (I) est ainsi obtenu avec un rendement global de 30 % à partir du nitrile de formule (III), une pureté chimique supérieure à 98% et une pureté énantiomérique supérieure à 99%.

La réduction du nitrile de formule (III) peut par exemple être effectuée par hydrogénation catalytique, de préférence catalysée par le nickel de Raney, plus préférentiellement dans un solvant alcoolique ammoniacal tel que le méthanol ammoniacal ou l'éthanol ammoniacal.
Elle peut également être effectuée par réduction chimique, par exemple par le borane complexé au tétrahydrofurane ou par le borohydrure de sodium/ acide trifluoroacétique.

La réaction de l'amine optiquement active de formule (VIII) avec le chloroformiate d'éthyle est préférentiellement effectuée en présence de triéthylamine ou de soude.

La réduction du carbamate de formule (IX) est préférentiellement effectuée à l'aide d'hydrure d'aluminium tel que l'hydrure de lithium aluminium ou l'hydrure de bis (2-méthoxy-éthoxy) sodium aluminum (RedAl®)

Les composés de formule (I) obtenus selon le procédé de la présente invention sont particulièrement utiles comme intermédiaires de synthèse dans la synthèse de l'ivabradine, de ses sels d'addition à un acide pharmaceutiquement acceptable et de ses hydrates.

L'exemple ci-dessous illustre l'invention.

### EXEMPLE : (1S)-4,5-Diméthoxy-1-(méthylaminométhyl)-benzocyclobutane, chlorhydrate :

### StadeA : 4,5-Diméthoxy-1-(aminométhyl)-benzocyclobutane :

Dans un hydrogénateur, charger le 4,5-diméthoxy-1-cyano-benzocyclobutane (6 kg), le méthanol (30 l), le nickel de Raney (0,6 l) et l'ammoniac (7,32 kg). Après avoir purgé par l'azote puis l'hydrogène, hydrogéner à 60°C sous 30 bars jusqu'à absorption de la quantité théorique d'hydrogène. Après retour à 20°C, filtrer le catalyseur, le rincer par le méthanol puis évaporer le solvant. On récupère le produit du titre sous la forme d'une huile, avec un rendement quantitatif.

### Stade B : Sel d'addition du (1S)-4,5-diméthoxy-1-(aminométhyl)-benzocyclobutane avec l'acide N-acétyl-L-glutamique :

Dans un réacteur, charger le composé obtenu au stade précédent (10 kg), l'acide N-acétyl-L-glutamique (9,79 kg) et l'éthanol 95% (200 l). Chauffer au reflux sous agitation puis revenir à 20°C. Laisser agiter une nuit à 20°C, filtrer le précipité et le laver avec de l'éthanol à 95%.

### Recristallisation :

Dans un réacteur, charger le sel obtenu précédemment et l'éthanol 95% (80 l). Chauffer au reflux sous agitation puis revenir à 20°C. Laisser agiter une nuit à 20°C, filtrer le précipité, le laver avec de l'éthanol à 95% et le sécher.

On récupère le produit du titre sous la forme d'un solide, avec un rendement de 40% à partir du composé obtenu au stade A et une pureté chimique et énantiomérique supérieure à 99%.

### Stade C : (1S)-4,5-Diméthoxy-1-(aminométhyl)-benzocyclobutane :

Dans un réacteur, charger sous agitation le composé obtenu au stade précédent (10 kg), l'eau (23 l), le dichlorométhane (37 l) et la solution aqueuse d'hydroxyde de sodium 3,25 N (23 l).
Après réaction à 20°C pendant 30 mn, la phase aqueuse est extraite par le dichlorométhane. La phase organique est séchée puis mise à sec. On récupère le produit attendu sous la forme d'une huile, avec un rendement de 98%.

### Stade D : (1S)-4,5-Diméthoxy-1-(éthoxycarbonylaminométhyl)-benzocyclobutane :

Dans un réacteur sous azote, charger le composé obtenu au stade précédent (10 kg) et le dichlorométhane (100 l). Couler successivement la triéthylamine (6,17 kg et le chloroformiate d'éthyle (5,49 l). Lorsque toute la matière première est consommée, laver le milieu réactionnel par de l'eau (2x 80 l) et une solution aqueuse 1N d'acide chlorhydrique (2x 80 l). Sécher la phase organique, puis la mettre à sec. Le solide obtenu est séché, pour conduire au produit du titre.

### Stade E : (1S)-4,5-Diméthoxy-1-(méthylaminométhyl)-benzocyclobutane :

Dans un réacteur, charger sous azote l'hydrure de lithium et d'aluminium (1,41 kg), et du tétrahydrofurane (32,5 l), puis couler à 20°C une solution du composé obtenu au stade précédent (5 kg) dans le tétrahydrofurane (50 l). Chauffer au reflux pendant 1 heure, puis refroidir à une température inférieure à 15°C pour hydrolyser le mélange réactionnel par de l'eau (1 l), une solution aqueuse 5 N d'hydroxyde de sodium (1 l), puis de l'eau (1 l). Filtrer le solide obtenu. Mettre à sec la phase organique. On récupère le produit du titre sous la forme d'une huile, avec un rendement de 93%.

### Stade F : (1S)-4,5-Diméthoxy-1-(méthylaminométhyl)-benzocyclobutane, chlorhydrate :

Dans un réacteur, charger le composé obtenu au stade précédent (5 kg), l'acétate d'éthyle (40 l) et l'éthanol (10 l). Agiter à 20°C pendant 30 mn, puis additionner par la vanne de fond du réacteur ou par un tube plongeant de l'acide chlorhydrique gazeux (1,012 kg). La suspension obtenue est agitée à 15-20°C pendant 1h, puis filtrée ou essorée. Le précipité est lavé par un mélange acétate d'éthyle/ éthanol 4/1 (2 x 5 l), puis séché, pour conduire au produit du titre avec un rendement de 92%.

## Revendications

1. Procédé de dédoublement de l'amine de formule (IV) : 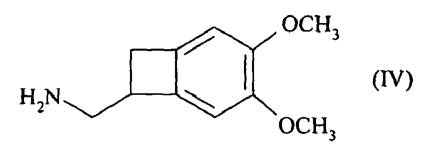 par réaction avec l'acide N-acétyl-L-glutamique,
suivie de la filtration ou de l'essorage de la suspension ainsi obtenue,
pour conduire au composé de formule (VII) : 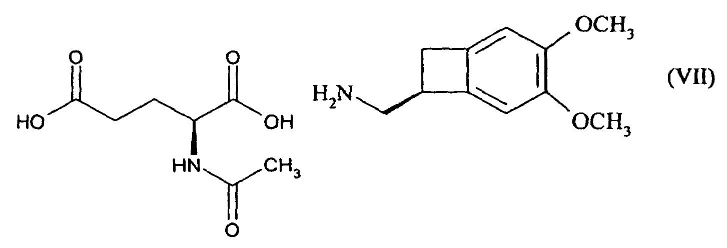 que l'on met en réaction avec une base, pour conduire à l'amine correspondante de formule (VIII), de configuration (S) :

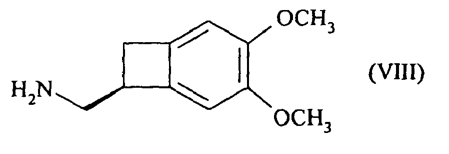

2. Procédé selon la revendication 1, dans lequel la réaction de l'amine de formule (IV) avec l'acide N-acétyl-L-glutamique est effectuée dans un solvant organique, seul ou en mélange avec un autre solvant organique, avec de l'eau ou avec un autre solvant organique et de l'eau.

3. Procédé selon la revendication 2, dans lequel la réaction de l'amine de formule (IV) avec l'acide N-acétyl-L-glutamique est effectuée dans un solvant alcoolique, un mélange d'un solvant alcoolique et d'eau, un mélange d'un solvant alcoolique et d'un autre solvant organique ou un mélange d'un solvant alcoolique, d'un autre solvant organique et d'eau.

4. Procédé selon l'une quelconque des revendications 1 à 3, dans lequel la base utilisée pour transformer le sel de formule (VII) en amine de formule (VIII) est la soude.

5. Procédé selon l'une quelconque des revendications 1 à 4, dans lequel le sel de formule (VII) est recristallisé une ou plusieurs fois dans un solvant organique, seul ou en mélange avec un autre solvant organique, avec de l'eau ou avec un autre solvant organique et de l'eau, avant sa transformation en composé de formule (VIII).

6. Procédé selon la revendication 5, dans lequel le sel de formule (VII) est recristallisé une ou plusieurs fois dans un solvant alcoolique, un mélange d'un solvant alcoolique et d'eau, un mélange d'un solvant alcoolique et d'un autre solvant organique, ou un mélange d'un solvant alcoolique, d'un autre solvant organique et d'eau, avant sa transformation en composé de formule (VIII).

7. Procédé selon l'une quelconque des revendications 1 à 6, dans lequel l'amine est dédoublée dans un mélange d'éthanol et d'eau ou dans un mélange d'acétate d'éthyle, d'éthanol et d'eau.

8. Procédé selon l'une quelconque des revendications 6 ou 7, dans lequel le sel de formule (VII) est recristallisé une ou plusieurs fois dans un mélange d'éthanol et d'eau ou dans un mélange d'acétate d'éthyle, d'éthanol et d'eau.

9. Procédé de synthèse du composé de formule (I), de configuration (S) : 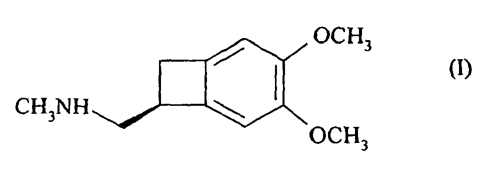 à partir du composé de formule (III) : 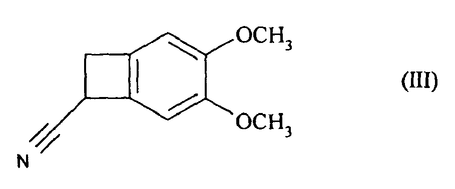 que l'on réduit,
pour conduire à l'amine racémique de formule (IV) : 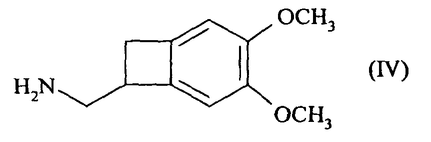 que l'on dédouble selon le procédé de l'une quelconque des revendications 1 à 8, pour conduire à l'amine optiquement active de formule (VIII) : 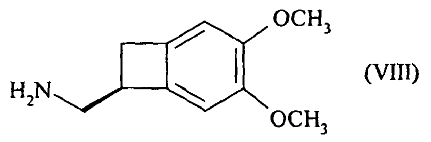 que l'on met en réaction avec le chloroformiate d'éthyle, pour conduire au composé optiquement actif de formule (IX) : 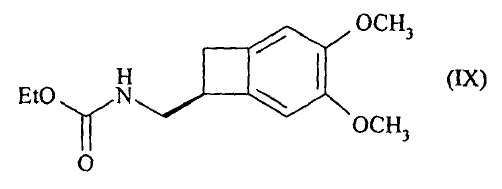 dont on réduit la fonction carbamate,
pour conduire au composé de formule (1).

10. Procédé selon la revendication 9, dans lequel le composé de formule (III) est réduit par hydrogénation catalysée par le nickel de Raney, ou par réaction avec le borane complexé au tétrahydrofurane ou avec le borohydrure de sodium/acide trifluoroacétique.

11. Procédé selon l'une quelconque des revendications 9 ou 10, dans lequel la réaction de l'amine de formule (VIII) avec le chloroformiate d'éthyle est effectuée en présence de triéthylamine ou de soude.

12. Procédé selon l'une quelconque des revendications 9 à 11, dans lequel le carbamate de formule (IX) est réduit par l'hydrure de lithium aluminium ou par l'hydrure de bis(2-méthoxy-éthoxy)sodium aluminium.

13. Procédé de synthèse de l'ivabradine, de ses sels pharmaceutiquement acceptables et de ses hydrates, dans lequel le composé de formule (III) est transformé en intermédiaire de formule (I) suivant le procédé de la revendication 9, puis l'intermédiaire de formule (I) est transformé en ivabradine.

## Claims

1. Process for the resolution of the amine of formula (IV) : 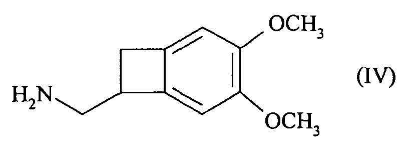 by reaction with N-acetyl-L-glutamic acid,
followed by filtration, or filtration under suction, of the suspension thereby obtained,
to yield the compound of formula (VII) : 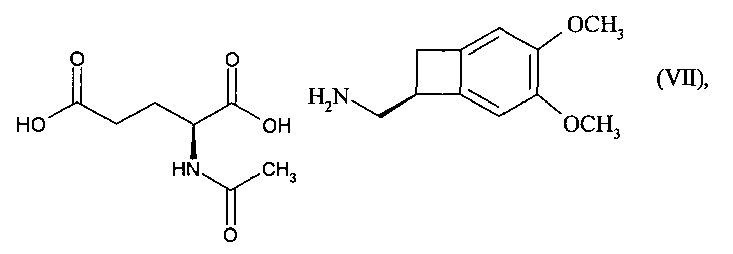 which is reacted with a base to yield the corresponding amine of formula (VIII) of configuration (S) :

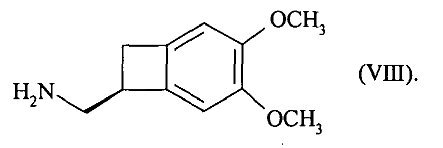

2. Process according to claim 1, wherein the reaction of the amine of formula (IV) with N-acetyl-L-glutamic acid is carried out in an organic solvent, alone or in admixture with another organic solvent, with water, or with another organic solvent and water.

3. Process according to claim 2, wherein the reaction of the amine of formula (IV) with N-acetyl-L-glutamic acid is carried out in an alcoholic solvent, a mixture of an alcoholic solvent and water, a mixture of an alcoholic solvent and another organic solvent, or a mixture of an alcoholic solvent, another organic solvent and water.

4. Process according to any one of claims 1 to 3, wherein the base used for converting the salt of formula (VII) into the amine of formula (VIII) is sodium hydroxide.

5. Process according to any one of claims 1 to 4, wherein the salt of formula (VII) is recrystallised once or more than once from an organic solvent, alone or in admixture with another organic solvent, with water, or with another organic solvent and water, before being converted into the compound of formula (VIII).

6. Process according to claim 5, wherein the salt of formula (VII) is recrystallised once or more than once from an alcoholic solvent, a mixture of an alcoholic solvent and water, a mixture of an alcoholic solvent and another organic solvent, or a mixture of an alcoholic solvent, another organic solvent and water, before being converted into the compound of formula (VIII).

7. Process according to any one of claims 1 to 6, wherein the amine is resolved in a mixture of ethanol and water or in a mixture of ethyl acetate, ethanol and water.

8. Process according to either claim 6 or claim 7, wherein the salt of formula (VII) is recrystallised once or more than once from a mixture of ethanol and water or from a mixture of ethyl acetate, ethanol and water.

9. Process for the synthesis of the compound of formula (I) of configuration (S) : 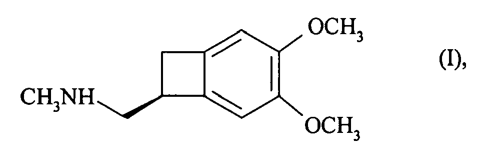 starting from the compound of formula (III) : 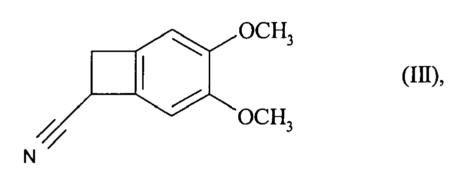 which is reduced
to yield the racemic amine of formula (IV) : 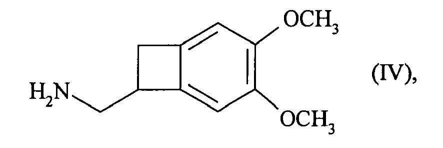 which is resolved according to the process of any one of claims 1 to 8 to yield the optically active amine of formula (VIII) : 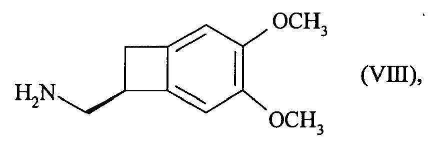 which is reacted with ethyl chloroformate to yield the optically active compound of formula (IX) : 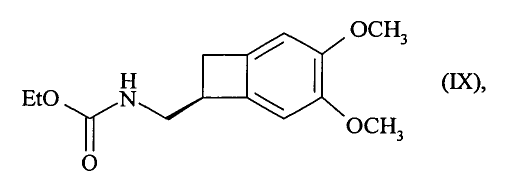 the carbamate function of which is reduced
to yield the compound of formula (I).

10. Process according to claim 9, wherein the compound of formula (III) is reduced by hydrogenation catalysed by Raney nickel or by reaction with borane complexed with tetrahydrofuran or reaction with sodium borohydride/trifluoroacetic acid.

11. Process according to either claim 9 or claim 10, wherein the reaction of the amine of formula (VIII) with ethyl chloroformate is carried out in the presence of triethylamine or sodium hydroxide.

12. Process according to any one of claims 9 to 11, wherein the carbamate of formula (IX) is reduced using lithium aluminium hydride or using sodium bis(2-methoxy-ethoxy)aluminium hydride.

13. Process for the synthesis of ivabradine, pharmaceutically acceptable salts thereof and hydrates thereof, wherein the compound of formula (III) is converted into the intermediate of formula (I) in accordance with the process of claim 9, and then the intermediate of formula (I) is converted into ivabradine.

## Patentansprüche

1. Verfahren zur Racematspaltung des Amins der Formel (IV): 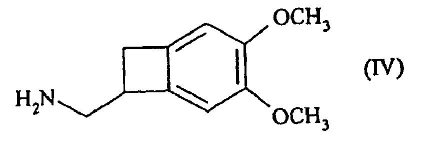 durch Umsetzen mit N-Acetyl-L-glutaminsäure,
gefolgt von einer Filtration oder dem Absaugen der in dieser Weise erhaltenen Suspension,
zur Bildung der Verbindung der Formel (VII): 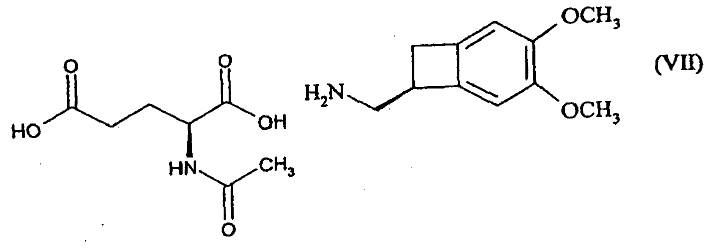 welche man mit einer Base umsetzt zur Bildung des entsprechenden Amins der Formel (VIII) in der Konfiguration (S):

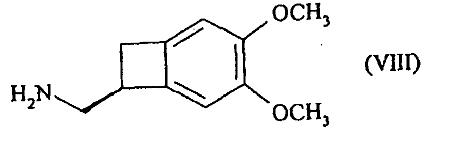

2. Verfahren nach Anspruch 1, worin die Umsetzung des Amins der Formel (IV) mit der N-Acetyl-L-glutaminsäure in einem organischen Lösungsmittel, allein oder in Mischung mit einem weiteren organischen Lösungsmittel, mit Wasser oder mit einem anderen organischen Lösungsmittel und Wasser durchgeführt wird.

3. Verfahren nach Anspruch 2, worin die Reaktion des Amins der Formel (IV) mit der N-Acetyl-L-glutaminsäure in einem alkoholischen Lösungsmittel, einer Mischung eines alkoholischen Lösungsmittels und Wasser, einer Mischung eines alkoholischen Lösungsmittels und eines weiteren organischen Lösungsmittels oder einer Mischung aus einem alkoholischen Lösungsmittel und einem weiteren organischen Lösungsmittel und Wasser durchgeführt wird.

4. Verfahren nach einem der Ansprüche 1 bis 3, worin die für die Umwandlung des Salzes der Formel (VII) in das Amin der Formel (VIII) verwendete Base Natriumhydroxid ist.

5. Verfahren nach einem der Ansprüche 1 bis 4, worin das Salz der Formel (VII) ein- oder mehrfach in einem organischen Lösungsmittel, allein oder in Mischung mit einem weiteren organischen Lösungsmittel, mit Wasser oder mit einem weiteren organischen Lösungsmittel und Wasser umkristallisiert wird, bevor es in die Verbindung der Formel (VIII) umgewandelt wird.

6. Verfahren nach Anspruch 5, worin das Salz der Formel (VII) ein- oder mehrfach in einem alkoholischen Lösungsmittel, einer Mischung aus einem alkoholischen Lösungsmittel und Wasser, einer Mischung aus einem alkoholischen Lösungsmittel und einem weiteren organischen Lösungsmittel oder einer Mischung aus einem alkoholischen Lösungsmittel und einem weiteren organischen Lösungsmittel und Wasser umkristallisiert wird, bevor es in die Verbindung der Formel (VIII) umgewandelt wird.

7. Verfahren nach einem der Ansprüche 1 bis 6, worin das Amin in einer Mischung aus Ethanol und Wasser oder einer Mischung aus Ethylacetat, Ethanol und Wasser in die optischen Isomeren aufgespalten wird.

8. Verfahren nach einem der Ansprüche 6 oder 7, worin das Salz der Formel (VII) ein- oder mehrfach in einer Mischung aus Ethanol und Wasser oder in einer Mischung aus Ethylacetat, Ethanol und Wasser umkristallisiert wird.

9. Verfahren zur Synthese der Verbindung der Formel (I) in der Konfiguration (S): 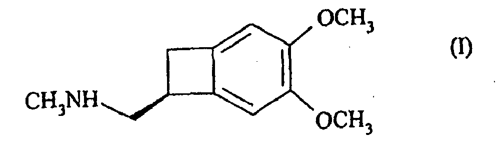 ausgehend von der Verbindung der Formel (III): 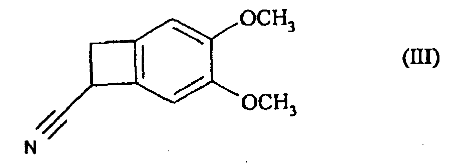 welche man reduziert zur Bildung des racemischen Amins der Formel (IV): 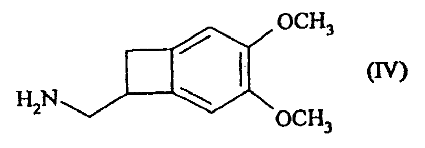 welches man nach dem Verfahren gemäß einem der Ansprüche 1 bis 8 in seine optischen Isomeren aufspaltet zur Bildung des optisch aktiven Amins der Formel (VI-II): 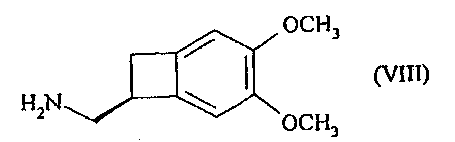 welches man mit Chlorameisensäureethylester umsetzt zur Bildung der optisch aktiven Verbindung der Formel (IX): 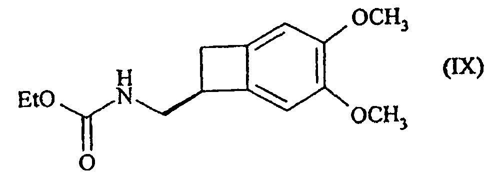 von welcher Verbindung man die Carbamatfunktion reduziert
zur Bildung der Verbindung der Formel (I).

10. Verfahren nach Anspruch 9, worin die Verbindung der Formel (III) durch mit Raney-Nickel katalysierte Hydrierung oder durch Reaktion mit dem Boran-Tetrahydrofuran-Komplex oder mit Natriumborhydrid/Trifluoressigsäure reduziert wird.

11. Verfahren nach einem der Ansprüche 9 oder 10, worin die Reaktion des Amins der Formel (VIII) mit Chlorameisensäureethylester in Gegenwart von Triethylamin oder Natriumhydroxid durchgeführt wird.

12. Verfahren nach einem der Ansprüche 9 bis 11, worin das Carbamat der Formel (IX) mit Lithiumaluminiumhydrid oder mit Bis(2-methoxy-ethoxy)-natriumaluminiumhydrid reduziert wird.

13. Verfahren zur Synthese von Ivabradin, seinen pharmazeutisch annehmbaren Salzen und seinen Hydraten, gemäß dem die Verbindung der Formel (III) nach dem Verfahren gemäß Anspruch 9 in das Zwischenprodukt der Formel (I) umgewandelt wird, wonach das Zwischenprodukt der Formel (I) in Ivabradin umgewandelt wird.
